# EUROPEAN PATENT APPLICATION

(11) **EP 1 477 110 A1**
(43) Date of publication of application: **17.11.2004**
(21) Application number: 04252821.6
(22) Date of filing: 14.05.2004
(51) Int. Cl.: A61B 5/00, A61B 5/02

(54) **Living body information measuring system and method of identifying a living body information detecting device currently used**

(30) Priority: 15.05.2003 JP 2003136900; 16.04.2004 JP 2004121689
(71) Applicant: SEIKO INSTRUMENTS INC., Chiba-shi, Chiba-ken 261 (JP)
(72) Inventor: Iijima, Ryuji, c/o Seiko Instruments Inc., Chiba-shi Chiba (JP); Moriya, Koichi, c/o Seiko Instruments Inc., Chiba-shi Chiba (JP)
(74) Representative: Sturt, Clifford Mark

(57) **Abstract**

A living body information measuring system, which is capable of accurately determining the originator of living body information data transmitted from living body information detecting devices used by multiple users, is disclosed.

A living body information detecting device (104,110,116,119) currently used by a user is identified by a portable device (102,105,109,114,120) worn on the user and having a first identification code associated with the user in advance. Then, information on the kind of the living body information detecting device is added to the first identification code to generate a second identification code, and the second identification code is transmitted (106,112,115,121) to the living body information detecting device. The living body information detecting device uses the second identification code as its own identification code and transmits it (108,113,118,122) to a data collector (101) together with the detected living body information data.

## Description

The present invention relates to a living body information measuring system that detects a condition of a user, a method of identifying a living body information detecting device currently used, a recording medium recording the method of identifying the living body information detecting device currently used, a portable device, and a data collector.

Systems have been disclosed in which sensors that detect living body information such as pulse rate or body temperature are installed in a bed or a toilet stool as living body information detecting devices, and living body information data are collected via communication from those living body information detecting devices and used for health care of individuals.

In above mentioned systems, the user of each living body information detecting device is identified as the living body information detecting device receives radio waves generated from an ID transmitter such as an ID card carried by the user of the living body information detecting device. In this case, when several people each carrying the ID transmitter are present near the living body information detecting device, the living body information detecting device may make erroneous judgment in identifying the user as it receives radio waves from the respective ID transmitters carried by the several people.

Further, it is necessary for the ID transmitter to keep transmitting the radio waves containing ID data periodically, leading to large electric current consumption by the ID transmitter.

Further, in the case where a data collector receives living body information data frommultiple types of living body information detecting devices, it is impossible to determine the living body information detecting device from which the living body information data has been received.

The present invention is made in view of the above circumstances, and provides a living body information measuring system including: a living body information detecting device that detects living body information data with a sensor and transmits the detected living body information data via radio communication; and a portable device carried by a user and provided with a first identification code associated with the user in advance, the portable device comprising: detecting device identifying means for identifying the living body information detecting device currently used by the user; identification code generating means for generating a second identification code for identifying the living body information detecting device; and identification code transmitting means for transmitting the second identification code by radio to the living body information detecting device currently used by the user.

Further, the living body information measuring system includes a data collector that collects living body information data via radio communication from a plurality of the living body information detecting devices, and the living body information detecting device transmits by radio the second identification code received from the portable device to the data collector, as a temporary identification code currently used by the user and together with the living body information data.

The living body information measuring system may be implemented in any one of the following configurations:
a configuration in which the portable device includes radio field intensity detecting means as the detecting device identifying means, the portable device identifying, as the living body information detecting device currently used by the user, the living body information detecting device having the highest radio field intensity as detected by the radio field intensity detecting means, and transmitting the second identification code to the identified living body information detecting device by the identification code transmitting means;
a configuration in which the portable device includes, as the detecting device identifying means, transmission power adjusting means for adjusting an output to the living body information detecting device, and reception sensitivity adjusting means, the portable device identifying, as the living body information detecting device currently used by the user, the living body information detecting device that has returned a response that can be received with the least reception sensitivity with respect to a transmission with the least transmission power, and transmitting the second identification code to the identified living body information detecting device by the identification code transmitting means; and
a configuration in which the portable device includes living body information detecting means as the detecting device identifying means, the portable device identifying the living body information detecting device as the living body information detecting device currently used by the user if there is a match in characteristics between living body information data detected by the portable device and living body information data detected by the living body information detecting device, and transmitting the second identification code to the identified living body information detecting device by the identification code transmitting means.

Further, another configuration may also be adopted in which the living body information detecting device judges whether a match is found between characteristics of living body information data detected by the portable device and those of living body information data detected by the living body information detecting device, and when the match is found, makes a transfer request for the second identification code to the portable device, and in which the portable device identifies as the living body information detecting device currently used by the user the living body information detecting device that has transmitted the transfer request for the second identification code, and transmits the second identification code to the living body information detecting device by the identified identification code transmitting means.

Living body information data detected by the portable device includes a pulse or acceleration of the user.

Further, the livingbody information detecting device includes identification code requesting means for requesting the portable device to transfer the second identification code when the living body information detecting device has started detection of living body information data.

Further, the living body information detecting device includes stop command transmitting means for transmitting a command for stopping detection of living body information data by the portable device when the living body information detecting device has started detection of living body information data.

The living body information detecting device includes start command transmitting means for transmitting a command for starting detection of living body information to the portable device when the living body information detecting device subsequently becomes unable to detect living body information.

Further, when unable to detect living body information, the living body information detecting device requests the portable device to transfer living body information data, and when unable to detect even the living body information data from the portable device, the living body information detecting device judges that an abnormality has occurred and performs emergency communication of the abnormality to the data collector. Further, when the living body information data has been normally received from the portable device, the living body information detecting device judges that a user has moved away from the living body information detecting device.

The identification code generating means generates the second identification code containing information indicative of a kind of the living body information detecting device based on the first identification code provided to the portable device.

As described above, with the living body information measuring system of the present invention, the wireless portable device worn on the user and previously provided with the first identification code associated with the user identifies the living body information detecting device currently used by the user, and transmits the second identification code, which is generated by adding information on the kind of the living body information detecting device to the first identification code of the portable device, to the living body information detecting device by radio. Then, the living body information detecting device uses the received second identification code as a temporary identification of the living body information detecting device and transmits the identification code to the data collector together with the living body information data. Accordingly, it is not necessary to establish association between each living body information detecting device and its user each time the user of the living body information detecting device changes. Further, it is possible to accurately determine the originator of living body information data transmitted from living body information detecting devices used by multiple users.

Further, the portable device identifies the living body information detecting device currently used by the user by the detecting device identifying means, and transmits the second identification code to that living body information detecting device by the identification code transmitting means. Accordingly, it is possible to transmit the second identification code only to the living body information detecting device used by a user, whereby no erroneous judgment occurs in identifying the living body information detecting device used by the user.

Further, when having started detection of living body information, the living body information detecting device requests, by using the identification code requesting means, the portable device carried by the user to transfer the second identification code. Thus, the identification code of the living body information detecting device can be regarded as the identification code corresponding to the user from the time when it is started to be used by the user. The portable device thus does not need to continue transmission of the identification code; normally, the portable device needs only to perform reception thereof. In radio communication, transmission operation generally involves less power consumption than reception operation and hence power saving can be achieved.

When the portable device includes the living body information detecting means, the living body information detecting device receives the second identification code from the portable device and then transmits, by the stop command transmitting means, the command for stopping detection of living body information with the portable device, thereby achieving saving of power consumption by the portable device.

When unable to detect living body information, the living body information detecting device transmits, by the start command transmitting means, the command for starting detection of living body information to the portable device. As a result, it is possible to detect living body information without a hitch even when the user has moved away from the living body information detecting device or an abnormality occurs.

At this time, the living body information detecting device requests the portable device to transfer living body information data. When unable to detect living body information from the portable device as well, the living body information detecting device judges by the judging means that an abnormality has occurred, and performs emergency communication of the abnormality to the data collector. Further, if the living body information data from the above-mentioned portable device is normal, the judging means judges that the user has moved away from the living body information detecting device. This allows the user to make an accurate judgment whether the living body information detecting device is no longer used or an abnormality has occurred.

Further, the portable device generates a second identification code containing information on the kindof the living body information detecting device, on the basis of the first identification code provided to the portable device and associated with the user in advance. The living body information detecting device designates the second identification code as a temporary identification code of the living body information detecting device. Accordingly, the identification code of the living body information detecting device can be regarded as the identification code corresponding to the user. Further, the kind of the living body information detecting device can be determined solely on the basis of the identification code thereof.

Embodiments of the invention will now be described by way of further example only and with reference to the accompanying drawings, in which:
Fig. 1 is a diagram schematically illustrating a living body information measuring system according to the present invention;
Fig. 2 is a functional block diagram of the present invention;
Fig. 3 is a diagram showing a hardware configuration of a controller of each of bed-type and toilet-type living body information detecting devices according to the present invention;
Fig. 4 is a diagram showing a hardware configuration of a control portion of a behavior monitor according to the present invention;
Fig. 5 is a diagram showing a hardware configuration of a data collector according to the present invention;
Fig. 6 is a diagram showing a packet structure of data transmitted from the living body information detecting device according to the present invention;
Fig. 7 is a diagram showing a hardware configuration of a portable device according to a first embodiment of the present invention;
Fig. 8 is a flowchart showing operations of a detection device identifying means in the portable device according to the first embodiment of the present invention;
Fig. 9 is a flowchart showing operations of a second-identification-code generating means in the portable device according to the present invention;
Fig. 10 is a flowchart showing operations on the living body information detecting device side corresponding to the detecting device identifying means in the portable device according to the first embodiment of the present invention;
Fig. 11 is a diagram showing a hardware configuration of a portable device according to a second embodiment of the present invention;
Fig. 12 is a flowchart showing operations of the detection device identifying means in the portable device according to the second embodiment of the present invention;
Fig. 13 is a diagram showing a hardware configuration of a portable device according to a third embodiment of the present invention;
Fig. 14 is a flowchart showing operations of the detecting device identifying means in the portable device according to the third embodiment of the present invention;
Fig. 15 is a flowchart showing operations of the bed-type device or the toilet-type device corresponding to the detecting device identifying means in the portable device according to the third embodiment of the present invention;
Fig. 16 is a flowchart showing operations of the behavior monitor corresponding to the detecting device identifying means in the portable device according to the third embodiment of the present invention; and
Fig. 17 is a diagram showing a hardware configuration of a control portion of a glucose monitor according to the present invention.

Hereinbelow, the present invention is describedwith reference to the drawings. It is to be noted that the present invention is not limited to the following embodiments.

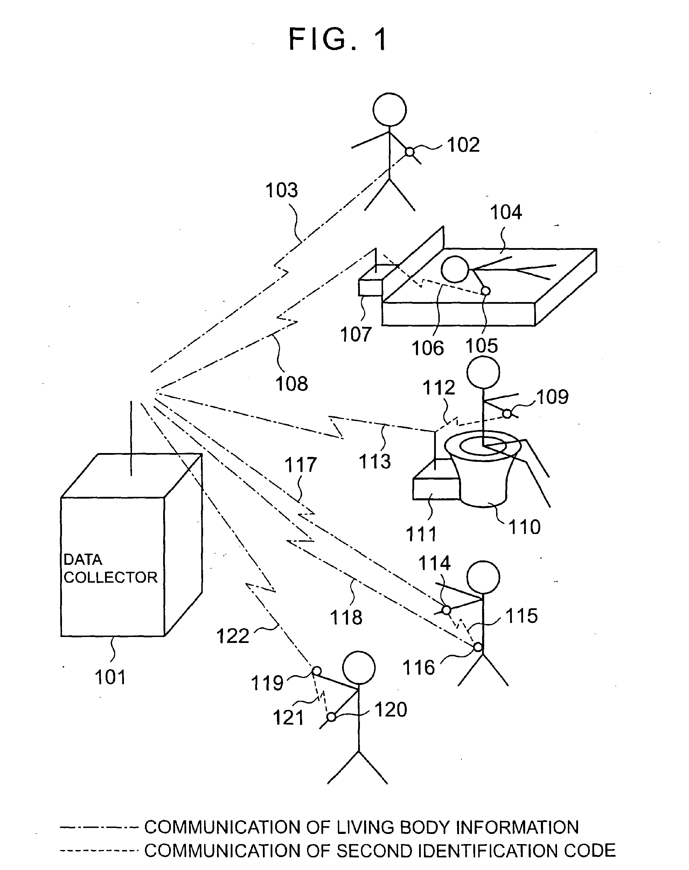

Fig. 1 schematically illustrates a living body information measuring system according to the present invention.

The living body information measuring system of the present invention includes a data collector 101 and portable devices 102, 105, 109, 114, and 120 carried by users, and a bed-type device 104, a toilet-type device 110, a behavior monitor 116, and a glucose monitor 119 that serve as living body information detecting devices.

The data collector 101 collects living body information data transmitted by radio from each living body information detecting device.

The portable devices 102, 105, 109, 114, and 120 each possess a first identification code associated with its user. In addition, the portable devices 102, 105, 109, 114, and 120 respectively have a radio transmission/reception function and a living body information detecting function, enabling those devices to perform radio transmission of living body information data to the data collector through living body information communication (103, 117).

The bed-type device 104 detects living body information of auserwho is lying down. Thebed-type device 104, which is controlled by a controller 107 having a radio communication function, receives a second identification code through radio communication from the portable device 105 worn on a user who is lying down (106), and performs radio transmission of the detected living body information data together with its identification code to the data collector through living body information communication (108).

The toilet-type device 110 detects living body information of a user who is sitting on the toilet stool. The toilet-type device 110, which is controlled by a controller 111 having a radio communication function, receives a second identification code through radio communication from the portable device 109 worn on a user who is sitting down (112), and performs radio transmission of the detected living body information data together with its identification code to the data collector through living body information communication (113).

The behavior monitor 116 is worn on the user to detect the acceleration and angular velocity related to the behaviors of the user. The behavior monitor 116, which has a radio communication function, receives a second identification code through radio communication from the portable device 114 worn on the user (115), and performs radio transmission of the detected living body information data together with its identification code to the data collector through living body information communication (118).

The glucose monitor 119 detects glucose in the blood of the user. The glucose monitor 119, which has a radio communication function, receives a second identification code through radio communication from the portable device 120 worn on the user (121), and performs radio transmission of the detected living body information data together with its identification code to the data collector through living body information communication (122).

The portable devices 102, 105, 109, and 114 may be implemented as wrist-watch type devices, card type devices, or the like.

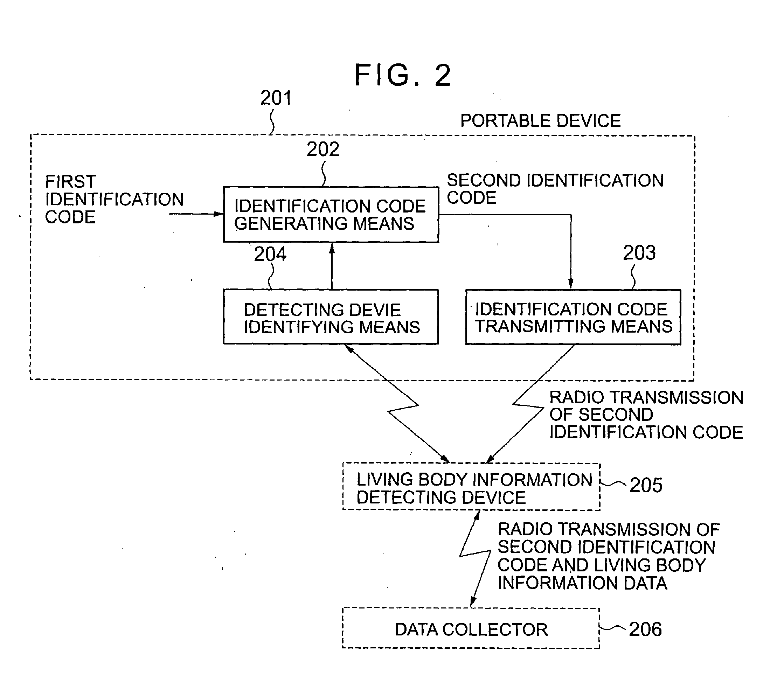

Fig. 2 is a block diagram illustrating functions with which a portable device 201 and a living body information detecting device 205 have. The portable device 201 possesses a first identification code associated with the user who is carrying the portable device 201. A detecting device identifying means 204 identifies the living body information detecting device that is currently used, by performing radio communication with the living body information detecting device 205. An identification code generating means 202 generates the second identification code based on information from the detecting device identifying means 204. An identification code transmitting means 203 transmits the second identification code to the living body information detecting device 205. The living body information detecting device 205 uses the received second identification code as its identification code and transmits it by radio to a data collector 206 together with the detected living body information data.

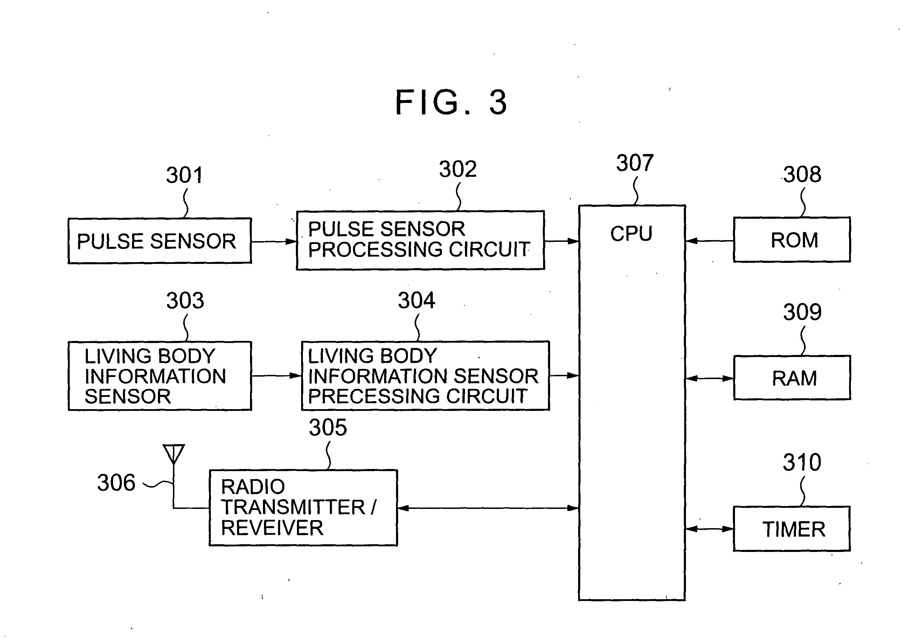

Fig. 3 shows the hardware configuration of a controller of each of bed-type and toilet-type living body information detecting devices. The respective controllers of the bed-type and toilet-type living body information detecting devices are of the same hardware configuration except for sensors that detect living body information.

A pulse sensor 301 detects the pulse of a user who is lying down in the bed when implemented as a bed-type device, and detects the pulse of a user who is sitting on the toilet stool when implemented as a toilet-type device. A signal detected by the pulse sensor 301 is converted into digital numerical data in a pulse sensor processing circuit 302, read by a CPU 307, and temporarily stored in a RAM 309.

Further, a living body information sensor 303, which collectively represents other living body information sensors, detects living body information of a user who is lying down in the bed when implemented as a bed-type device, and detects living body information of a user who is sitting on the toilet stool when implemented as a toilet-type device. A signal detected by the living body information sensor 303 is converted into digital numerical data in a living body information sensor processing circuit 304 and read by the CPU 307. Multiple kinds of sensors are mounted as the living body information detecting sensors; a respiration sensor, a body temperature sensor, and the like are mounted in the case of a bed-type device, and a body temperature sensor, a urine sugar sensor, and the like are mounted in the case of a toilet-type device.

A timer 310 can have its time set and read by the CPU 307.

The CPU 307 controls the whole living body information detecting device in accordance with software stored in a ROM 308. The CPU 307 reads out living body information from the sensors at a fixed time interval, and temporarily stores the data thus obtained in the RAM 309 together with the reading-out time. In addition, the CPU 307 transfers by radio to the data collector the living body information data read out by the living body information sensor and temporarily stored in the RAM 309, by using a radio transmitter/receiver 305 and an antenna 306, or receives the second identification code from the above-mentioned portable device.

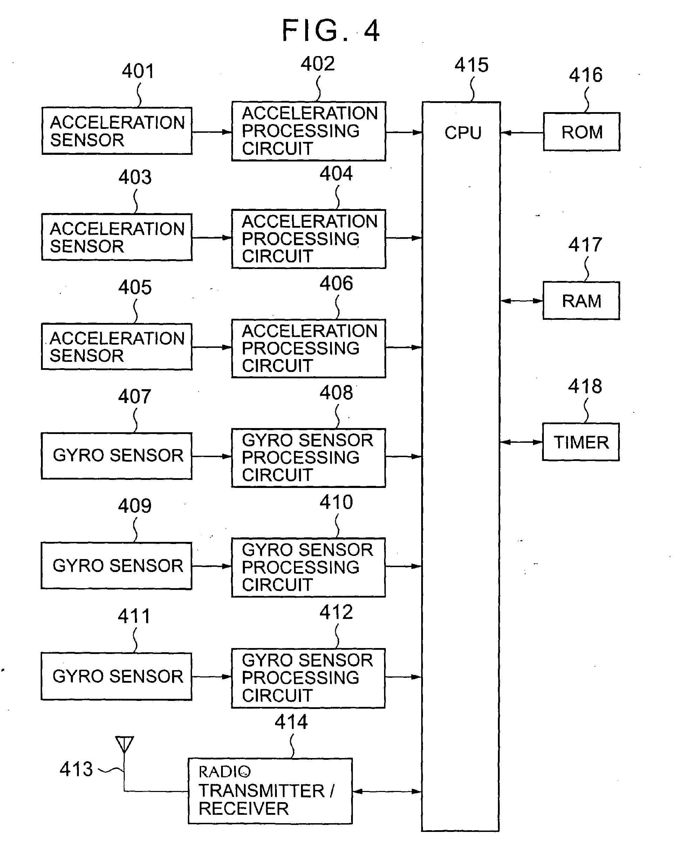

Fig. 4 shows the hardware configuration of a control portion of the behavior monitor in the living body information detecting device.

The behavior monitor is a device that is worn on a user to detect the behaviors of the user by detecting an acceleration, an angular velocity, etc.

Three acceleration sensors 401, 403, and 405 are used to detect the accelerations in three axial directions of the user. Signals detected by the three acceleration sensors 401, 403, and 405 are each converted into digital numerical data in acceleration processing circuits 402, 404, and 406, respectively, to be read by a CPU 415 and temporarily stored in a RAM 417.

Further, three gyro sensors 407, 409, and 411 are used to detect the angular speeds in three axial directions of the user. Signals detectedby the three gyro sensors 407, 409, and 411 are each converted into digital numerical data in gyro sensor processing circuits 408, 410, and 412, respectively, to be read by the CPU 415 and temporarily stored in the RAM 417.

While acceleration sensors and gyro sensors are used as the sensors in this example, a GPS and the like may be used as the sensors other than those.

A timer 418 can have its time set and read by the CPU 415.

The CPU 415 controls the whole behavior sensor in accordance with software stored in a ROM 416. The CPU 415 reads out living body information from the respective sensors at a fixed time interval, and temporarily stores the data thus obtained in the RAM 417 together with the reading-out time. In addition, the CPU 415 transfers by radio to the data collector the living body information data read out by the respective sensors and temporarily stored in the RAM 417, by using a radio transmitter/receiver 414 and an antenna 413, or receives the second identification code from the above-mentioned portable device.

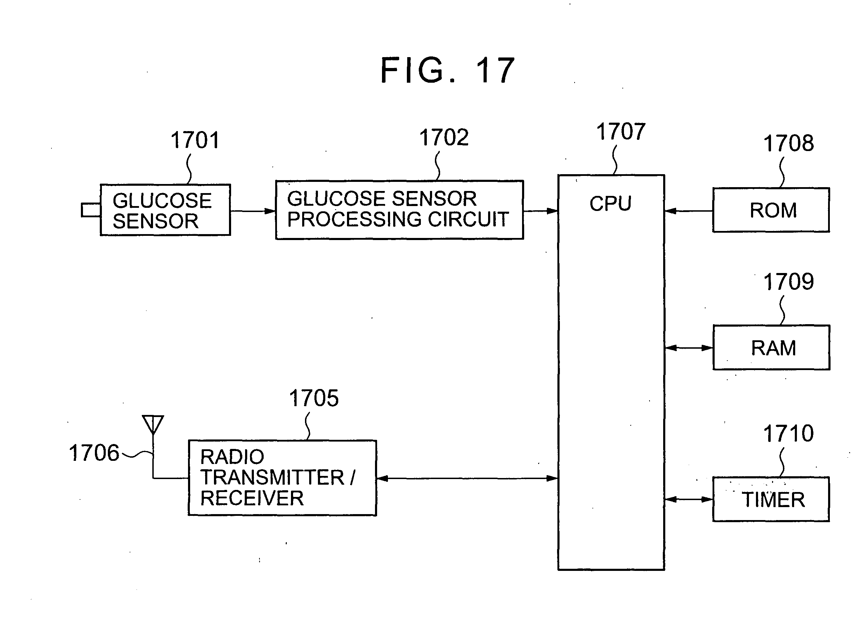

Fig. 17 shows the hardware configuration of a control portion of the glucose monitor in the living body information detecting device.

A glucose sensor 1701 detects glucose in the blood sucked in by using a blood-sucking needle. The glucose sensor detects glucose concentration by utilizing the fact that when a voltage is applied to blood that has been treated in a prescribed manner, the value of a current flowing in the blood is proportional to glucose concentration. Of course, the method of detecting glucose concentration is not limited to this.

A signal detected by the glucose sensor is converted into digital numerical data in a glucose sensor processing circuit 1702, read by a CPU 1707, and temporarily stored in a RAM 1709.

A timer 1710 can have its time set and read out by the CPU 1707.

The CPU 1707 controls the glucose monitor in accordance with software stored in a ROM 1708. The CPU 1707 temporarily stores in the RAM 1709 the time at which the glucose in blood was detected, together with living body information data. In addition, the CPU 1707 transfers by radio to the data collector the living body information data read out by the glucose sensor and temporarily stored in the RAM 1709, by using a radio transmitter/receiver 1705 and an antenna 1706, or receives the second identification code from the above-mentioned portable device.

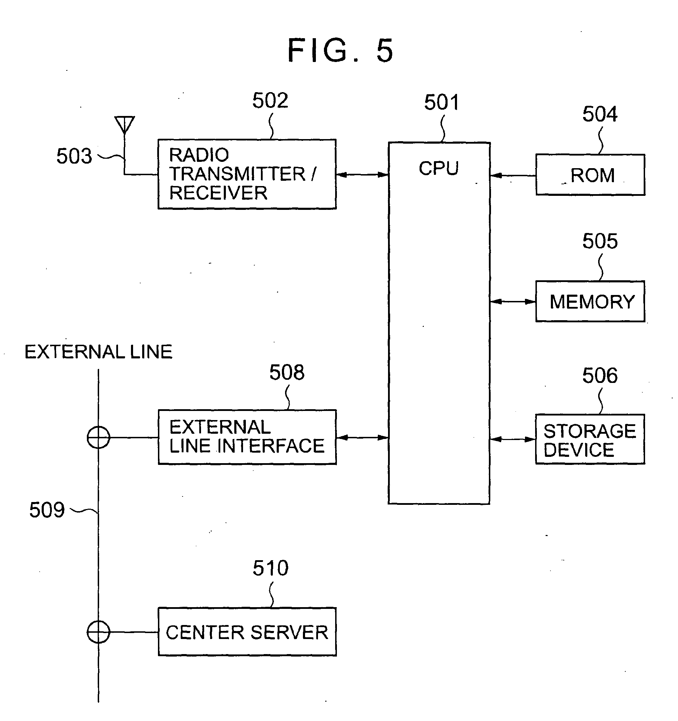

Fig. 5 shows the hardware configuration of the data collector. The data collector is controlled by a CPU 501 operating in accordance with a program stored in a ROM 504. By using a radio transmitter/receiver 502 and an antenna 503, the CPU 501 transmits instruction commands to the living body information detecting device or receives data sent from the living body information detecting device. Data received from the living body information detecting device is temporarily stored in a memory 505 before being stored into a storage device 506. A hard disk or the like is used as the storage device 506.

The data collector includes an external line interface 508 and connects to an external line 509, for transmitting collected data to the external. Examples of the external line include a LAN and a public line, and the data collector connects to a central server 510 via those lines.

The central server combines living body information data from the data collector to perform various analyses or to provide services for external use.

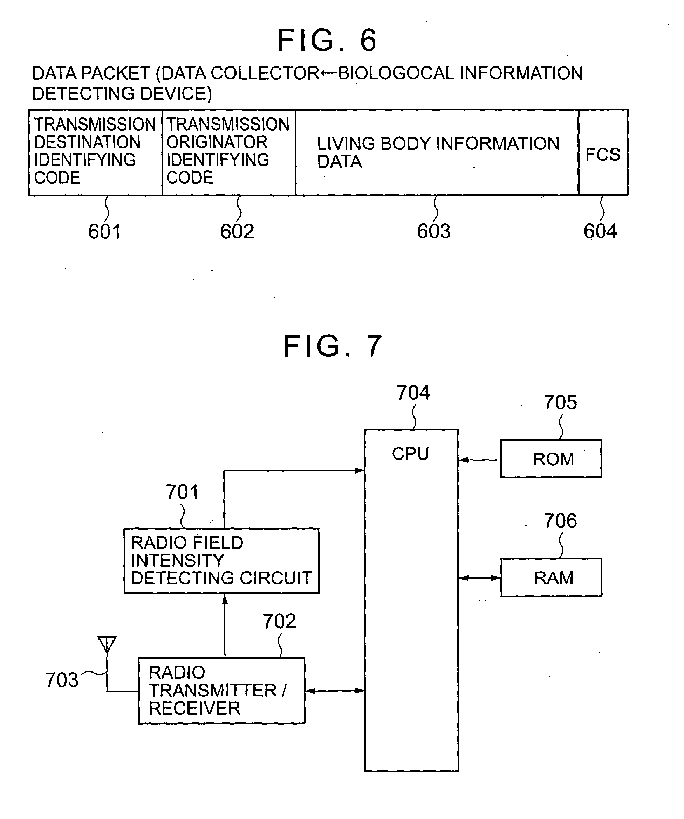

Fig. 6 is a view showing the packet structure of data transmitted by radio to the data collector from the living body information detecting device.

A packet consists of a transmission destination identifying code 601, a transmission originator identifying code 602, living body information data 603, and an FCS 604.

The transmission destination identifying code 601 is an identification code for identifying the data collector to which data is transmitted. The transmission originator identifying code 602 is an identification code for identifying the living body information detecting device from which data has been transferred. The second identification code received from the above-mentioned portable device is used as the transmission originator identifying code.

The living body information data 603 are data sampled with the living body information detecting device.

The FCS 604 is a code for detecting a packet error. As such a code, there is used a 16-bit CRC code or the like which is calculated using from the leading end of the packet to the end of the living body information data 603.

The data collector determines, from the transmission originator identifying code 602 contained in the packet, the living body information detecting device from which the received data has been transmitted, and determines whose living body information the data is.

### First embodiment

Fig. 7 shows the hardware configuration of the portable device mentioned above according to a first embodiment of the present invention.

A CPU 704 controls the whole portable device in accordance with software stored in a ROM 705. The detecting device identifying means for identifying the living body information detecting device currently used by the user and the identification code generating means for generating the second identification code for discriminating among the living body information detecting devices are also realizedby the above software. Thus, the operations carried out by the detecting device identifying means and the identification code generating means are operations executed by the CPU 704.

A radio transmitter/receiver 702 transmits/receives a message to/from the living body information detecting device existing in the periphery thereof via an antenna 703.

A radio field intensity detecting circuit 701 measures the intensity of radio waves transmitted from the living body information detecting device existing in the periphery thereof. The living body information detecting device that is currently used is identified from the results of this radio field intensity measurement.

A RAM 706 is used for temporary storage, etc. to store the intensity of the radio waves from the living body information detecting device existing in the periphery thereof.

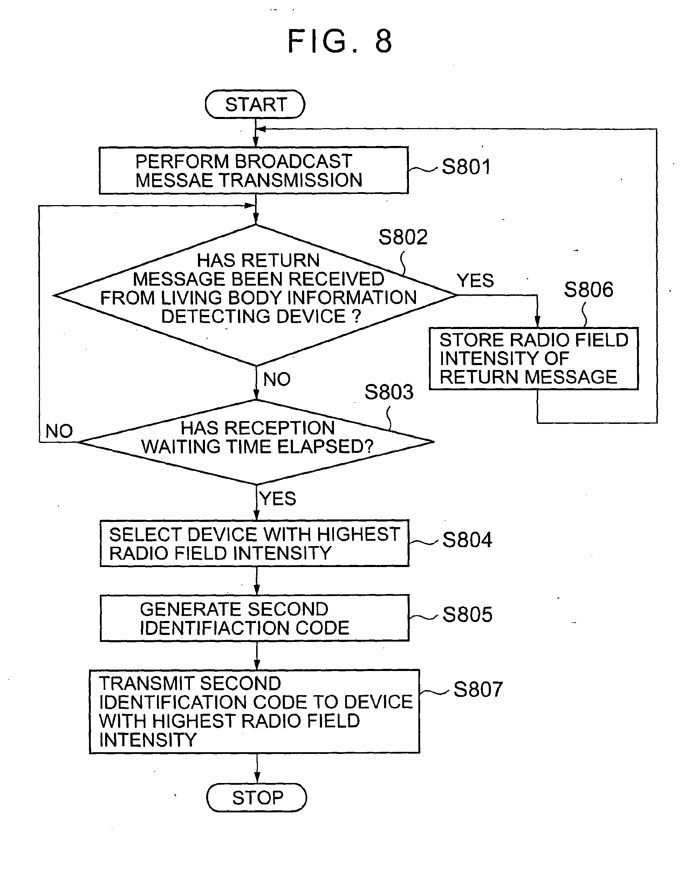

Fig. 8 is a flowchart showing operations of the detecting device identifying means for identifying the living body information detecting device currently used by means of the radio field intensity detecting circuit 701, in the portable device configured as shown in Fig. 7. The operations of the detecting device identifying means are executed by the CPU 704.

In step S801, the detecting device identifying means transmits a message by radio (through broadcast communication) to all the living body information detecting devices existing in the periphery thereof. The message is one requesting each living body information detecting device to return a message, and a code of several bytes or the like is used as its data format. The message returned by each living body information detecting device contains information on the kind and the like of the living body information detecting device.

In step S802, the detecting device identifying means makes a judgement as to whether it has successfully received the return message from each living body information detecting device. Then, just as it has received the return message, the detecting device identifying means detects the radio field intensity of the return message by the radio field intensity detecting circuit (701 in Fig. 7) . Ifthereturnmessagehasbeensuccessfullyreceived, theprocess advances to step S806 where the field intensity of the return message is stored in the RAM (706 in Fig. 7).

In the case where multiple living body information detecting devices are present in the periphery thereof, the detecting device identifying means receives multiple return messages. Accordingly, in step S803, the detecting device identifying means continues the reception of the return messages from the living body information detecting devices until a preset reception waiting time elapses.

If it is judged in step S803 that the preset reception waiting time has elapsed, the process advances to step S804 where the detecting device identifying means identifies as the currently used device the living information detecting device that has returned the message with the highest radio field intensity from among the radio field intensities of the return messages stored in step S806.

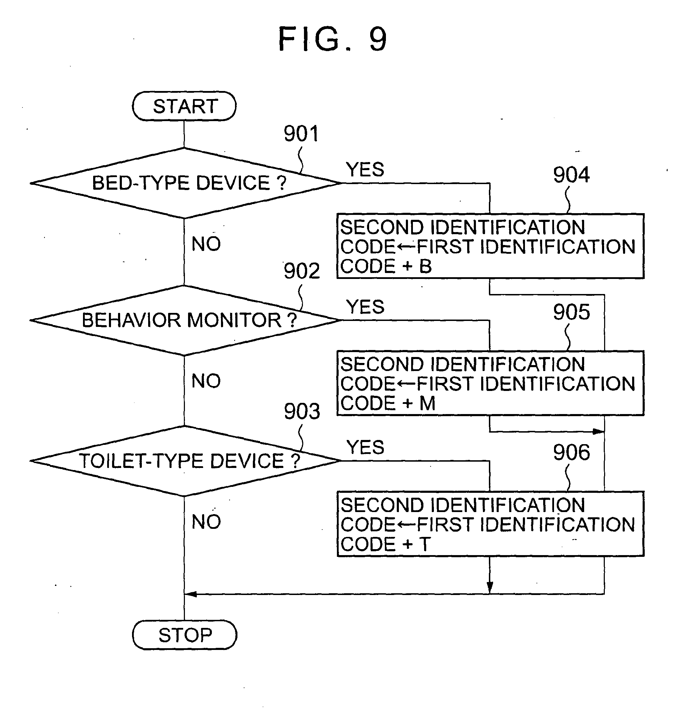

The operations of the detecting device identifying means executed by the CPU 704 are now complete. In the next step S805, the CPU 704 causes the identification code generating means to generate an identification code (second identification code) for the living body information detecting device identified-in step S804. Operations of the identification code generating means are described with reference to Fig. 9. In step S807, the second identification code generated in step S805 is transmitted to the living body information detecting device identified in step S804.

Fig. 9 is a flowchart showing operations performed by the second-identification-code generating means in step S805 of Fig. 8. The operations of the identification code generating means are executed by the CPU 704.

In step S901, the identification code generating means judges whether the living body information detecting device to which the second identification code is to be sent is the bed-type device. Since the return message received from the living body information detecting device in step S802 of Fig. 8 contains data for identifying the kind of the living body information detecting device, the above judgment is made by using this data.

If it is judged in step S901 that the living body information detecting device is the bed-type device, the process advances to step S904 where a code obtained by attaching the character "B", for example, to the identification code (first identification code) of the portable device is designated as the identification code (second identification code) of the bed-type livingbody information detecting device. For instance, when the identification code (first identification code) of the portable device is "1234", "1234B" is the identification code (second identification code) of the bed-type living body information detecting device.

In step S902, the identification code generating means judges whether the living body information detecting device to which the second identification code is to be sent is the behavior monitor. Since the return message received from the living body information detecting device in step S802 of Fig. 8 contains data for identifying the kind of the living body information detecting device, the above judgment is made by using this data.

If it is judged in step S902 that the living body information detecting device is the behavior monitor, the process advances to step S905 where a code obtained by attaching the character "M", for example, to the identification code (first identification code) of the portable device is designated as the identification code (second identification code) of the behavior monitor. For instance, when the identification code (first identification code) of the portable device is "1234", "1234M" is the identification code (second identification code) of the behavior monitor.

In step S903, the identification code generating means judges whether the living body information detecting device to which the second identification code is to be sent is the toilet-type device. Since the return message received from the living body information detecting device in step S802 of Fig. 8 contains data for identifying the kind of the living body information detecting device, the above judgment is made by using this data.

If it is judged in step S903 that the living body information detecting device is the toilet-type device, the process advances to step S906 where a code obtained by attaching the character "T", for example, to the identification code (first identification code) of the associated portable device is designated as the identification code (second identification code) of the toilet-type living body information detecting device. For instance, when the identification code (first identification code) of the portable device is "1234", "1234T" is the identification code (second identification code) of the toilet-type living body information detecting device.

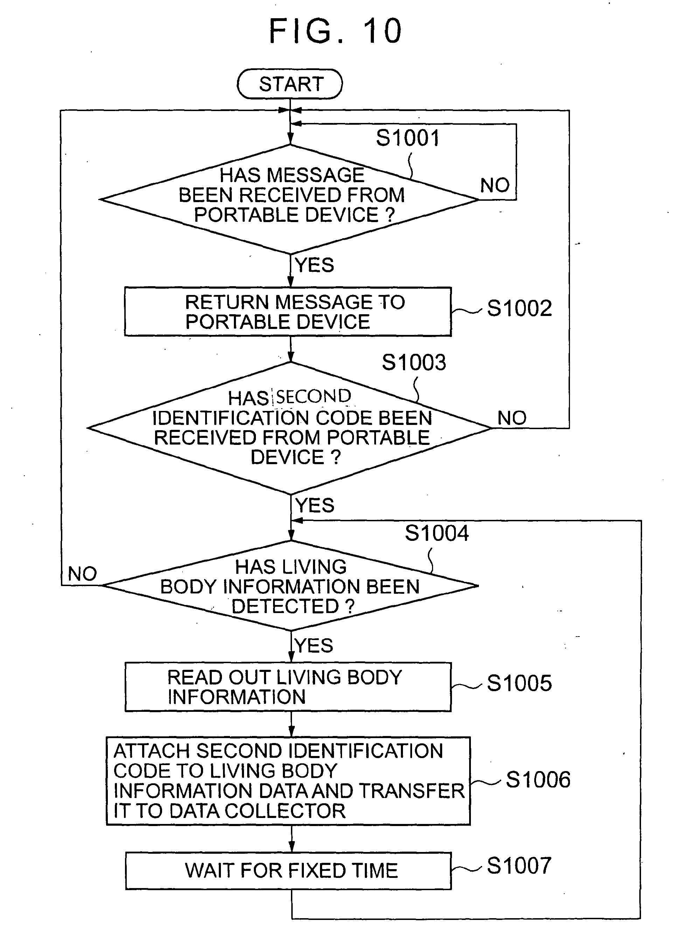

Fig. 10 is a flowchart illustrating operations of the living body information detecting device corresponding to the operations of the detecting device identifying means.

In step S1001, the living body information detecting device judges whether a message from the portable device has been received. This message is the one transmitted by the portable device in step 801 of Fig. 8. If the message has been received, in step S1002, the living body information detecting device transmits a return message to the portable device from which the message has been transmitted. The return message contains data indicative of the kind of the living body information detecting device. The portable device receives the return message, and the radio field intensity of the return message is measured and stored (step S806 of Fig. 8) .

Of the living body information detecting devices present in the periphery thereof, the portable device transmits to the living body information detecting device having the highest radio field intensity the identification code (second identification code) of that living body information detecting device. Thus, the living body information detecting device makes a judgment in step S1003 whether it has successfully received the second identification code. If the second identification code has not been successfully received, the process returns to step S1001 where the living body information detecting device again waits for reception of a message from the portable device. If the second identification code has been successfully received, the living body information detecting device designates the identification code as an identification code that the living body information detecting device will use from then on.

In step S1004, the living body information detecting device judges whether living body information can be detected from the living body information sensors. If the living body information cannot be detected, the living body information detecting device judges that the living body information detecting device is not actually used at present by the user, and the process returns to step S1001 where the living body information detecting device again waits for message from the portable device.

If the living body information can be detected in step S1004, in step S1005, the living body information detecting device reads out the living body information from the living body information sensors. Then in step S1006, the second identification code received in step S1003 is attached to the living body information data and the resultant data is transmitted by radio to the data collector.

In step 51007, the living body information detecting device is held inwaiting for a fixed period of time. Thereafter, the process returns to step 51004 where the living body information detecting device again repeats the reading out of the living body information data. The fixed period of time during which the living body information detecting device is held in waiting in step S1007 represents the interval for reading out the living body information data from the living body-information sensors.

### Second embodiment

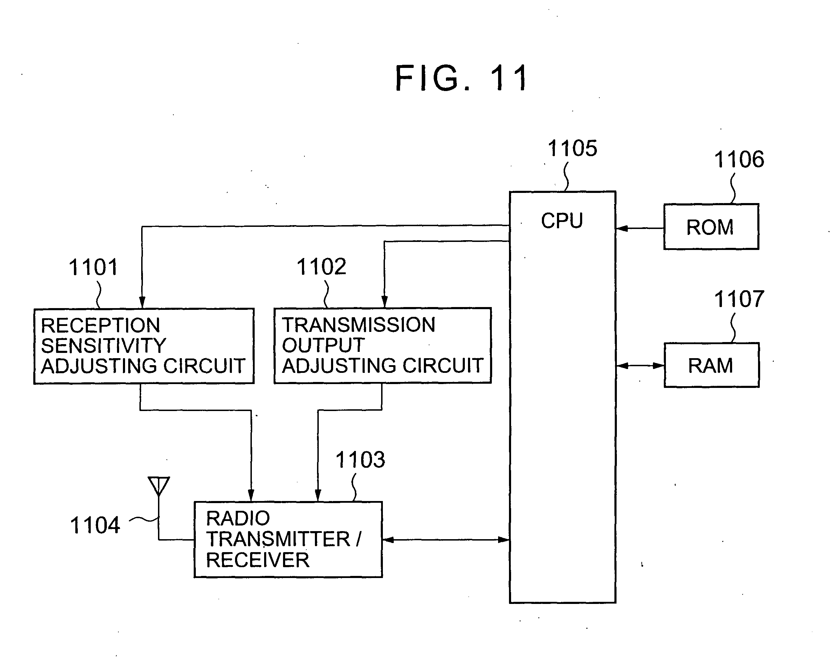

Fig. 11 shows the hardware configuration of the portable device according to a second embodiment of the present invention.

A CPU 1105 controls the whole portable device in accordance with software stored in a ROM 1106. The detecting device identifying means for identifying the living body information detecting device currently used by the user and the identification code generating means for generating the second identification code for discriminating among the living body information detecting devices are also realizedby the above software. Thus, the operations carried out by the detecting device identifying means and the identification code generating means are operations executed by the CPU 1105.

A RAM 1107 is used to store control data for the CPU 1105. A radio transmitter/receiver 1103 transmits/receives messages to/from the living body information detecting devices existing in the periphery thereof via an antenna 1104.

A transmission power adjusting circuit 1102 changes the transmission power of the radio transmitter/receiver 1103 upon command from the CPU 1105.

A reception sensitivity adjusting circuit 1101 changes the reception sensitivity of the radio transmitter/receiver 1103 upon command from the CPU 1105.

The CPU 1105 changes the transmission power and reception sensitivity of the radio transmitter/receiver 1103 by using the transmission power adjusting circuit 1102 and the reception sensitivity adjusting circuit 1101, respectively, to identify the living body information detecting device currently used.

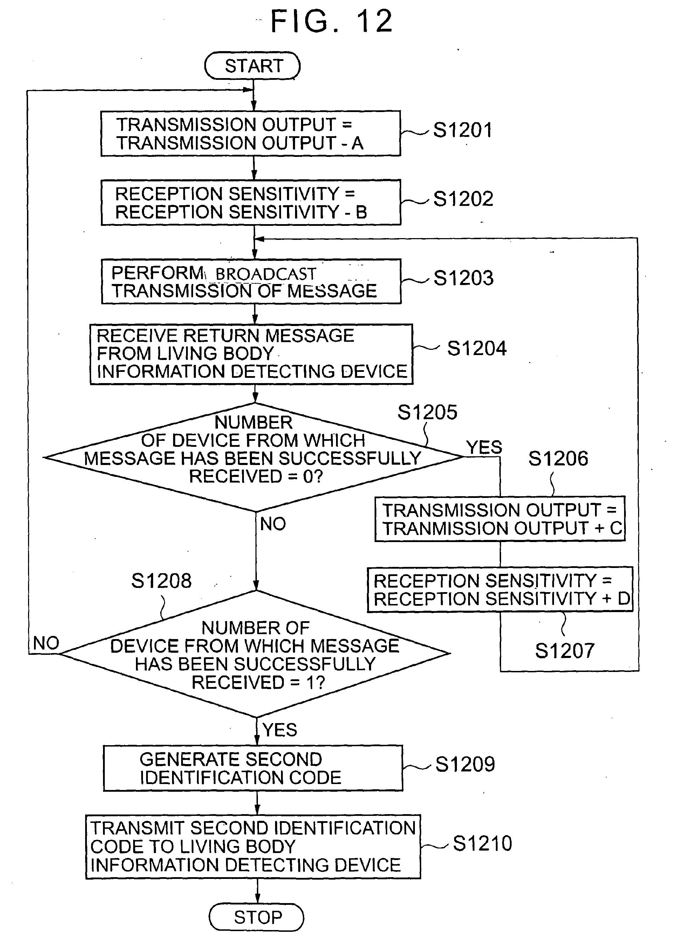

Fig. 12 is a flowchart illustrating operations of the detecting device identifying means for identifying the living body information detecting device currently used, in the portable device configured as shown in Fig. 11. The operations of the detecting device identifying means are executed by the CPU 1105.

In step S1201, the detecting device identifying means sets the transmission power to a value reduced by a preset value A. In step S1202, the detecting device identifyingmeans sets the reception sensitivity to a value reduced by a preset value B.

In step S1203, the detectingdevice identifyingmeans transmits a message by radio (through broadcast communication) to all the living body information detecting devices existing in the periphery thereof. The message is one requesting each living body information detecting device to return a message, and a code of several bytes or the like is used as its data format. The message returned by each living body information detecting device contains information on the kind and the like of the living body information detecting device.

In step 1204, the detecting device identifying means receives the return message from the living body information detecting device.

In step S1205, the detecting device identifying means judges whether it has successfully received the message from the living body information detecting device. If the message has not been successfully received in step S1205, the detecting device identifying means judges that the transmission power and the reception sensitivity are too low, and hence sets the transmission power to a value increased by a preset value C and sets the reception sensitivity to a value increased by a preset value D, respectively in steps S1206 and S1207. Then the process advances to step S1203 where transmission of the message is performed again.

If the return message from the living body information detecting device has been successfully received in step 51205, the process advances to step 51208 where the detecting device identifying means judges whether the number of the living body information detecting devices from which it has successfully received the return message is one. If it is judged in step S1208 that there are more than one living body information detecting devices from which it has successfully received the return message, the process returns to step S1201, and after making the transmission power and the reception sensitivity lower in steps S1201 and 51202, respectively, the detecting device identifying means again transmits a message to the living body information detecting devices present in the periphery thereof in step S1203.

If it is judged in step S1208 that the number of the living body information detecting devices from which the message has been successfully received is one, this may be regarded as indicating that the nearest of the living body information detecting devices has been detected. The detecting device identifying means identifies that living body information detecting device as the living body information detecting device that is currently used by the user.

The operations of the detecting device identifying means executed by the CPU 1105 are now complete. In the next step 51209, the CPU 1105 causes the identification code generating means to generate an identification code (second identification code) for the identified living body information detecting device. Then in step S1210, the CPU 1105 transmits the second identification code generated in step S1209 to the identified living body information detecting device. The operations of the identification code generating means are the same as those of Fig. 9.

In this embodiment, the operations performed by the living body information detecting device are the same as those shown in the flowchart of Fig. 10.

### Third embodiment

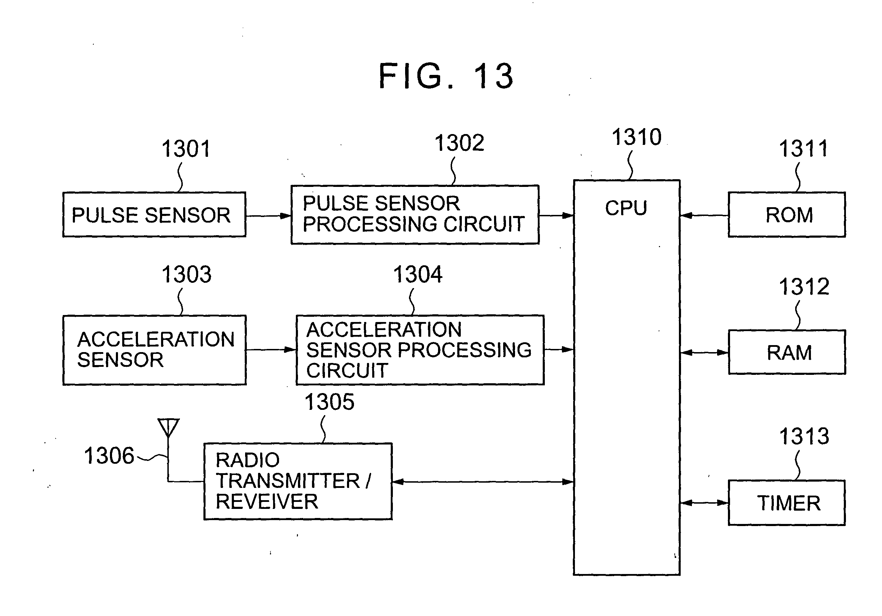

Fig. 13 shows the hardware configuration of the portable device according to a third embodiment of the present invention.

The portable device of this embodiment has a function of detecting living body information. Herein, the description is directed to a case where a pulse sensor 1301 that detects the pulse and an acceleration sensor 1303 that detects the acceleration of a user are used. Examples of other sensors used include those for detecting body temperature, blood flow, and the like.

The pulse wave detected by the pulse sensor 1301 is converted into digital data in apulse sensorprocessing circuit 1302. Further, the acceleration detected by the acceleration sensor 1303 is converted into digital data in an acceleration sensor processing circuit 1304. Those sensor information data are temporarily stored in a RAM 1312 by means of a program stored in a ROM 1311 and due to control by a CPU 1310 that controls the whole portable device.

The CPU 1310 controls the whole portable device in accordance with software stored in the ROM 1311.

By using a radio transmitter/receiver 1305 and an antenna 1306, the CPU 1310 transmits by radio the living body information data temporarily stored in the RAM 1312 to the data collector, exchanges information with the living body information detecting devices present in the periphery thereof, or transmits the second identification code.

The identification code generating means for generating the second identification code used to discriminate among the living body information detecting devices is also realized by the software. Thus, the operations carried out by the identification code generating means are operations executed by the CPU 1310.

With a timer 1313, the current time can be called up by the CPU 1310. When each sensor samples data, the CPU 1310 calls up the time indicated by the timer 1313 and sets it as the sampling time. The sampling time is stored together with the sampling data of each sensor.

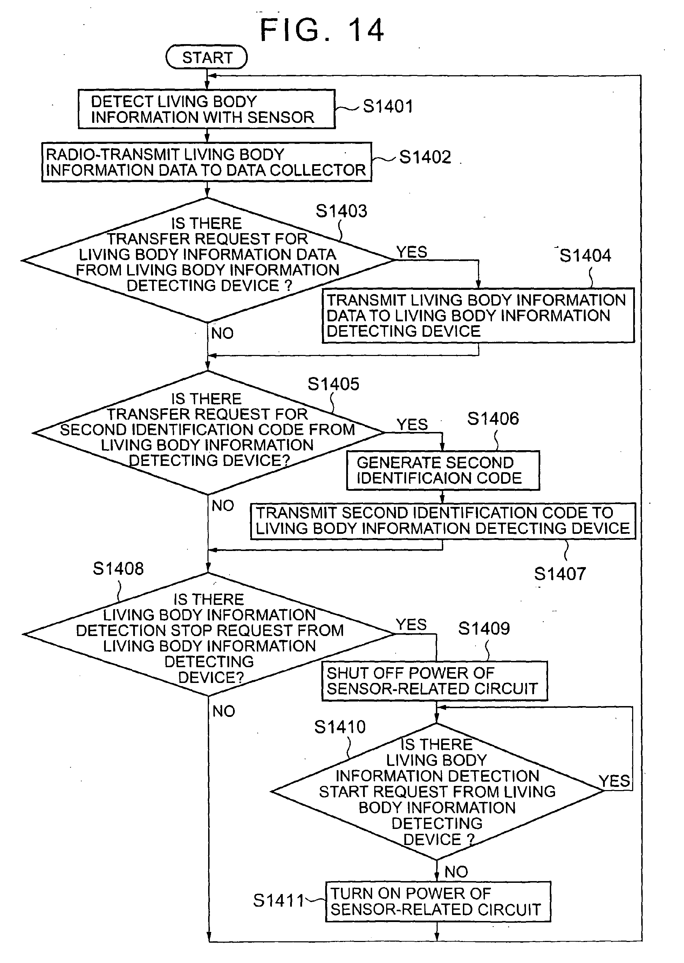

Fig. 14 is a flowchart illustrating operations of the portable device in the case where the living body information detecting device identifies the user by itself, in which the portable device generates and transmits the second identification code.

In step S1401, the portable device detects living body information with the pulse sensor, the acceleration sensor, and the like, and in step S1402, the portable device transmits the living body information data by radio to the data collector.

In step 51403, the portable device judges whether a transmission request for the living body information data has been made by the living body information detecting device located in the periphery thereof. When starting detection of living body information with its own sensor, the living body information detecting device located in the periphery requests the portable device located in the periphery thereof to transmit the living body information data detected on the portable device side. The transmission request for the living body information data mentioned above is made for both the pulse and the acceleration or individually for each of the pulse and acceleration.

If it is judged in step S1403 that there has been the transmission request for living body information data from the living body information detecting device present in the periphery, the process advances to step S1404 where the portable device transmits the living body information data to that living body information detecting device.

In step S1405, the portable device judges whether the living body information detecting device present in the periphery thereof has made a transmission request for the identification code (second identification code) of that living body information detecting device. Upon finding a match between the characteristics of the living body information data transmitted from the portable device and those of the living body information data detected by itself, the living body information detecting device in the periphery requests the portable device to transmit an identification code (second identification code) which the living body information detecting device will use from then on.

If it is judged in step S1405 that there has been a transmission request for the second identification code from the living body information detecting device present in the periphery, the process advances to step S1406 where the identification code generating means operated by the CPU 1310 of the portable device generates the identification code (second identification code) for the living body information detecting device. Then, in step S1407, the second identification code is transmitted to that living body information detecting device.

In step S1408, the portable device judges whether a living body information detection stop request has been made from the living body information detecting device to which the second identification code has been transmitted. After receiving the second identification code from the portable device, the living body information detecting device transmits the living body information detection stop request by radio to the portable device to achieve power saving for the portable device.

If it is judged in step S1408 that the portable device has received the living body information detection stop request, the power source of the sensor-related circuit is shut off to save power consumption in step S1409.

In step S1410, the portable device judges whether there has been a living body information detection start request from the living body information detecting device. When unable to detect living body information with its own sensors, the living body information detecting device transmits the living body information detection start request to the portable device. If the living body information detection start request has been made, the portable device turns on the power source of the sensor-related circuit in step 51411 so that the process returns to step S1401 to start detection of living body information again.

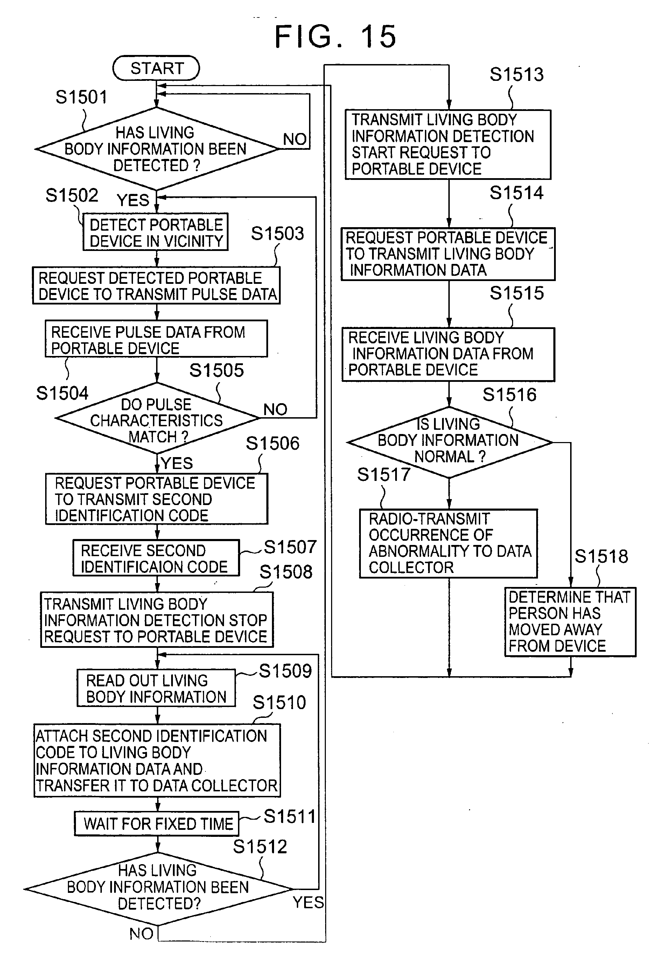

Fig. 15 is a flowchart showing operations of the bed-type or toilet-type living body information detecting device for identifying the user by itself and acquiring the second identification code from the portable device owned by the user.

In step S1501, the living body information detecting device judges whether its own living body information sensor has started detection ofliving bodyinformation. If the livingbody information sensor has started detection of living body information, in the case of the bed-type living body information detecting device, it is judged that the user has lain down on the bed; in the case of the toilet-type living body information detecting device, it is judged that the user has sat down on the toilet stool. Thus, the process advances to step S1502 where the living body information detecting device searches for a portable device present in the periphery thereof. Then, the living body information detecting device requests the portable device thus searched out in step S1503 to transmit pulse data detected by the portable device, and receives the pulse data from the portable device in step S1504.

In step S1505, the living body information detecting device judges whether there is a match between the characteristics of the pulse data detected by the living body information detecting device and those of the pulse data received from the portable device. In this case, the above-mentioned match between the characteristics of the respective pulse data is judged to have occurred when the difference between numerical values respectively detected by the living body information detecting device and the portable device, the numerical values each representing the characteristics of the pulse data, such as the pulse rate, cycle, phase, or the like, is smaller than a predetermined value.

If it is judged in step S1505 that the characteristics of the respective pulse data do not match, the process returns to step S1502 where the living body information detecting device makes a search for another portable device present in the periphery thereof.

If it is judged in step 51505 that there is a match between the characteristics of the respective pulse data, the-process advances to step S1506 where the living body information detecting device requests the portable device to transmit an identification code (second identification code) which the living body information detecting device will use from then on.

In step S1507, the living body information detecting device receives the second identification code from the portable device. Then, in step S1508, the living body information detecting device transmits a living body information detection stop request to the portable device. This is performed to save power of the portable device by detecting living body information on the bed-type device or toilet-type device side.

In step S1509, the living body information detecting device reads out living body information data from the living body information sensors such as the pulse sensor.

In step S1510, the living body information detecting device attaches the second identification code received in step S1507 to the living body information data thus read out, and transmits the resultant data to the data collector.

In step S1511, the living body information detecting device is held in waiting for a fixed period of time. The fixed period of time represents the sampling interval for detecting living body information.

In step S1512, the living body information detecting device checks whether it can detect living body information. If living body information can be detected, the process returns to step S1509 where the living body information detecting device reads out living body information data again.

If it is judged in step S1512 that living body information cannot be detected, this may mean that the subject has moved away from the bed or the toilet-type device or an abnormality has occurred. The process advances to step S1513 in such a case.

In step S1513, the living body information detecting device transmits a living body information detection start request to the portable device.

In step S1514, the living body information detecting device transmits to the portable device a transmission request for the living body information data such as the pulse rate detected by the portable device.

In step 51515, the living body information detecting device receives the living body information data from the portable device.

In step S1516, the living body information detecting device judges whether the living body information data such as pulse rate received from the portable device is normal. If the living body information data such as pulse rate is normal, the process advances to step S1518 where it is judged that the user has moved away from the bed-type device or the toilet-type device. If the living body information data such as pulse rate is abnormal, the process advances to step S1517 where the information on the occurrence of abnormality is transmitted by radio to the data collector.

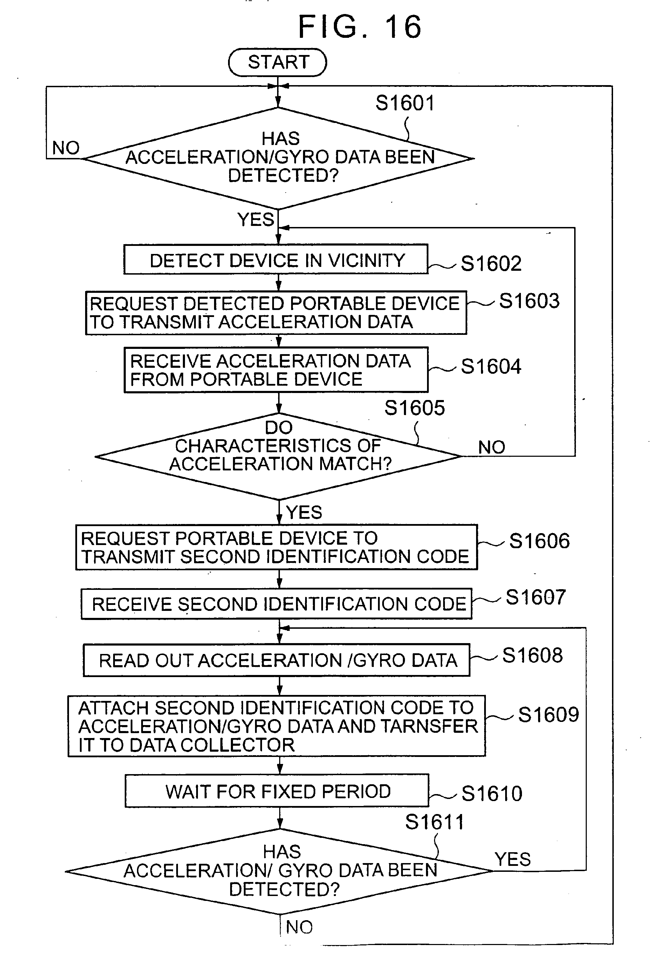

Fig. 16 is a flowchart illustrating operations of the behavior monitor for identifying the user by itself and acquiring the second identification code from the portable device owned by the user.

In step S1601, the behavior monitor judges whether the acceleration sensor or the gyro sensor has started detection of acceleration data or gyro data. If the detection of the acceleration data or gyro data has been started, it is possible to judge that the user has started using the behavior monitor. Thus, the process advances to step S1602 where the behavior monitor searches for a portable device present in the vicinity thereof. Then, the behavior monitor requests the portable device thus searched out in step S1603 to transmit the acceleration data detected by the portable device, and receives acceleration data form the portable device in step S1604.

In step S1605, the behavior monitor judges whether there is a match between the characteristics of the acceleration data detected by the behavior monitor and those of the acceleration data received from the portable device. In this case, the above match between the characteristics of the respective acceleration data is judged to have occurred when the difference between numerical values respectively detected by the behavior monitor and the portable device, the numerical values each representing the characteristics of the acceleration data, such as the acceleration, cycle, phase, or the like, is smaller than a predetermined value.

If it is judged in step S1605 that the characteristics of the respective acceleration data do not match, the process returns to step S1602 where the behavior monitor performs detection of another portable device present in the periphery thereof.

If it is judged in step S1605 that a match is found between the characteristics of the respective acceleration data, the process advances to step S1606 where the behavior monitor requests the portable device to transmit the second identification code.

In step S1607, the behavior monitor receives the second identification code from the portable device.

In step S1608, the behavior monitor reads out living body information data from the acceleration or gyro sensor.

In step S1609, the behavior monitor attaches the second identification code received in step S1607 to the living body information data thus read out, and transmits the resultant data to the data collector.

In step S1610, the behavior monitor is held in waiting for a fixed period of time. The fixed period of time represents the sampling interval for detecting data.

In step 51611, the behavior monitor checks whether it can detect the acceleration or gyro data. If the acceleration or gyro data can be detected, the process returns to step S1608 where the behavior monitor reads out the acceleration or gyro data again.

If the acceleration or gyro data cannot be read out in step S1608, the process returns to step S1601 to wait for the detection of the acceleration or gyro data.

While in the third embodiment the living body information detecting device receives living body information data from the portable device and compares the living body information data with the living body information data detected by its own sensors to thereby identify the user, the identification of the user may be performed by the portable device. In that case, the portable device receives living body information data from the living body information detecting device and compares the living body information data with the living body information data detected by the sensors of the portable device to thereby identify the user.

Note that a configuration is also possible in which the program installed in the above-described living body information detecting device for realizing the functions performed by the CPU is stored in a computer-readable recording medium, and the program recorded in the recording medium is read and executed by a device equipped with sensors and capable of reading and executing the program, thereby using the device as the living body information detecting device of the present invention.

Further, a configuration is also possible in which the program installed in the portable device for realizing the functions performed by the CPU is stored in a computer-readable recording medium, and the program recorded in the recording medium is read and executed by a device capable of reading and executing the program, thereby using the device as the portable device of the present invention. In this case, using a device equipped with sensors and capable of reading and executing the program enables identification of the user to be performed with the living body information detecting device as in the third embodiment of the present invention.

## Claims

1. A living body information measuring system comprising:
a living body information detecting device that detects living body information with a sensor and transmits data of the detected living body information via radio communication; and
a portable device carried by a user and provided with a first identification code associated with the user in advance, the portable device including: detecting device identifyingmeans for identifying the living body information detecting device currently used by the user; identification code generating means for generating a second identification code for identifying the living body information detecting device; and identification code transmitting means for transmitting the second identification code by radio to the living body information detecting device currently used by the user.

2. A living body information measuring system according to Claim 1, **characterized in that**:
the living body information measuring system comprises a data collector that collects living body information data via radio communication from a plurality of the living body information detecting devices; and
the living body information detecting device transmits by radio the second identification code received from the portable device to the data collector, as a temporary identification code currently used by the user and together with the living body information data.

3. A living body information measuring system according to Claim 1 or 2, **characterized in that** the portable device comprises radio field intensity detecting means as the detecting device identifying means, the portable device identifying, as the living body information detecting device currently used by the user, the living body information detecting device having the highest radio field intensity as detected by the radio field intensity detecting means, and transmitting the second identification code to the identified living body information detecting device by the identification code transmitting means.

4. A living body information measuring system according to Claim 1 or 2, **characterized in that** the portable device comprises, as the detecting device identifying means, transmission power adjustingmeans for adjusting an output to the livingbody information detecting device, and reception sensitivity adjusting means, the portable device identifying, as the living body information detecting device currently used by the user, the living body information detecting device that has returned a response that can be received with the least reception sensitivity with respect to a transmission with the least transmission power, and transmitting the second identification code to the identified living body information detecting deviceby the identification code transmitting means.

5. A living body information measuring system according to Claim 1 or 2, **characterized in that** the portable device comprises living body information detecting means as the detecting device identifying means, the portable device identifying the living body information detecting device as the living body information detecting device currently used by the user if there is a match in characteristics between living body information data detected by the portable device and living body information data detected by the living body information detecting device, and transmitting the second identification code to the identified living body information detecting device by the identification code transmitting means.

6. A living body information measuring system according to Claim 5, **characterized in that**:
the living body information detecting device receives living body information data from the portable device, and transmits a transfer request for the second identification code to the portable device if there is a match in characteristics between living body information data detected by the portable device and living body information data detected by the living body information detecting device; and
the portable device identifies, as the living body information detecting device currently used by the user, the living body information detecting device that has transmitted the transfer request for the second identification code, and transmits the second identification code to the living body information detecting device by the identification code transmitting means.

7. A living body information measuring system according to Claim 5 or 6, **characterized in that** the living body information data detectedby the portable device comprises a pulse or acceleration of the user.

8. A living body information measuring system according to any one of Claims 1 to 7, **characterized in that** the living body information detecting device comprises identification code requesting means for requesting the portable device to transfer the second identification code when the living body information detecting device has started detection of living body information data.

9. A living body information measuring system according to Claim 5 or 6, **characterized in that** the living body information detecting device includes stop command transmitting means for transmitting a command for stopping detection of living body information data by the portable device when the living body information detecting device has started detection of living body information data.

10. A living body information measuring system according to Claim 5 or 6, **characterized in that** the living body information detecting device includes start command transmitting means for transmitting a command for starting detection of living body information to the portable device when the living body information detecting device becomes unable to detect living body information.

11. A living body information measuring system according to Claim 5 or 6, wherein when unable to detect living body information, the living body information detecting device requests the portable device to transfer living body information data, and when unable to detect even the living body information data from the portable device, the living body information detecting device judges that an abnormality has occurred and performs emergency communication of the abnormality to the data collector.

12. A living body information measuring system according to Claim 5 or 6, wherein when unable to detect living body information, the living body information detecting device requests the portable device to transfer living body information data, and when the living body information data has been normally received from the portable device, the living body information detecting device judges that a user has moved away from the living body information detecting device.

13. A living body information measuring system according to Claim 1 or 2, **characterized in that** the identification code generating means generates the second identification code containing information indicative of a kind of the living body information detecting device based on the first identification code provided to the portable device.

14. A method of identifying a living body information detecting device currently used which is used for the living body information measuring system as claimed in Claim 1, the method. comprising the steps of: identifying the living body information detecting device currently used by a user; generating an identification code for identifying the living body information detecting device; and transmitting the identification code by radio to the living body information detecting device currently used by a user.

15. A method of identifying a living body information detecting device currently used according to Claim 14, **characterized in that** the step of transmitting the identification code by radio to the living body information detecting device currently used by the subject comprises transmitting the identification code as a temporary identification code currently used by the user and together with the living body information data to a data collector by radio.

16. A method of identifying a living body information detecting device currently used according to Claim 14, **characterized in that** the step of identifying the living body information detecting device currently used by the user comprises the steps of: detecting radio field intensities of living body information detecting devices in a vicinity; and selecting the living body information detecting device with the highest radio field intensity.

17. A method of identifying a living body information detecting device currently used according to Claim 14, **characterized in that** the step of identifying the living body information detecting device currently used by the user comprises the steps of: adjusting transmission power to living body information detecting devices in a vicinity; adjusting reception sensitivities to responses from the living body information detecting devices; and determining as the living body information detecting device currently used by the user the living body information detecting device that has returned, with respect to transmission with the least transmission power, a response that can be received with the least reception sensitivity.

18. A method of identifying a living body information detecting device currently used according to Claim 14, **characterized in that** the step of identifying the living body information detecting device currently used by the user comprises the steps of: detecting livingbody information data with a portable device; detecting living body information data with the living body information detecting devices; and comparing the living body information data from the portable device and the living body information data from the living body information detecting devices and determining, as the living body information detecting device currently used by the user, the living body information detecting device that has detected living body information data whose characteristics match the characteristics of the living body information data detected by the portable device.

19. A computer-readable recording medium storing a program for causing a computer to execute a method of identifying a living body information detecting device currently used which is used in the living body information measuring system as claimed in Claim 1, the method comprising the steps of: identifying the living body information detecting device currently used by a user; generating an identification code for identifying the living body information detecting device; and transmitting the identification code by radio to the living body information detecting device currently used by a user.

20. A portable device used in the living body information measuring system as claimed in Claim 1 and capable of communicating with a living body information detecting device, the living body information detecting device detecting living body information data with a sensor and transmitting the detected living body information data via radio communication, **characterized in that** the portable device is carried by a user, is provided with a first identification code associated with the user in advance, and comprises: detecting device identifyingmeans for identifying the living body information detecting device currently used by the user; identification code generating means for generating a second identification code for identifying the living body information detecting device; and identification code transmitting means for transmitting the second identification code by radio to the living body information detecting device.

21. A data collector which is used in the living body information measuring system as claimed in Claim 1 and collects living body information data from a living body information detecting device via radio communication, the living body information detecting device detecting living body information data with a sensor and transmitting the detected living body information data via radio communication, **characterized in that** the data collector receives an identification code for identifying the living body information detecting device as a temporary identification code currently used by a user and together with the living body information data by radio from the living body information detecting device.

22. A living body information detecting device used in the living body information measuring system as claimed in Claim 1, the living body information detecting device detecting living body information data with a sensor and transmitting the detected living body information data via radio communication, **characterized in that** the living body information detecting device transmits a second identification code received from a portable device carried by a user as a temporary identification code currently used by the user and together with the living body information data by radio to a data collector.
